(19) Europäisches Patentamt
European Patent Office
Office européen des brevets

(11) Veröffentlichungsnummer: **0 091 604**
**B1**

(12) **EUROPÄISCHE PATENTSCHRIFT**

(45) Veröffentlichungstag der Patentschrift: **16.10.85**

(51) Int. Cl.⁴: **C 07 C 51/58,** C 07 C 53/42, C 07 C 51/04, C 07 C 53/124

(21) Anmeldenummer: **83103096.0**

(22) Anmeldetag: **29.03.83**

(54) **Verfahren zur Herstellung von Isobuttersäurefluorid bzw. Isobuttersäure.**

(30) Priorität: **10.04.82 DE 3213395**

(43) Veröffentlichungstag der Anmeldung: **19.10.83 Patentblatt 83/42**

(45) Bekanntmachung des Hinweises auf die Patenterteilung: **16.10.85 Patentblatt 85/42**

(84) Benannte Vertragsstaaten: **DE FR GB IT NL**

(56) Entgegenhaltungen:
**EP - A - 0 031 886**
**GB - A - 2 092 142**
**GB - A - 2 099 821**

**Die Akte enthält technische Angaben, die nach dem Eingang der Anmeldung eingereicht wurden und die nicht in dieser Patentschrift enthalten sind.**

(73) Patentinhaber: **Röhm GmbH, Kirschenallee Postfach 4242, D-6100 Darmstadt 1 (DE)**

(72) Erfinder: **Besecke, Siegmund, Dr. Dipl.-Chem., Auf dem Kreuzberg 6, D-6104 Seeheim-Jugenheim (DE)**
Erfinder: **Schröder, Günter, Dr. Dipl.-Chem., Leipziger Strasse 7, D-6105 Ober-Ramstadt (DE)**
Erfinder: **Siegert, Hermann-Josef, Dr. Dipl.-Chem., Inselstrasse 21, D-6100 Darmstadt (DE)**

**EP 0 091 604 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

*Gebiet der Erfindung*

Die Erfindung betrifft die Herstellung von Isobuttersäurefluorid durch kontinuierliche Umsetzung von Propylen mit Kohlenmonoxid und Fluorwasserstoff sowie insbesondere die Verbindung dieses Verfahrens mit der weiteren Umsetzung des gebildeten Isobuttersäurefluorids zu Isobuttersäure. Die erste Reaktionsstufe entspricht der Koch'schen Carbonsäuresynthese mit überschüssigem Fluorwasserstoff als Katalysator. Das in der ersten Reaktionsstufe in Abwesenheit von Wasser erzeugte Isobuttersäurefluorid lässt sich in einer zweiten Stufe mit Wasser zu Fluorwasserstoff und Isobuttersäure hydrolysieren. Diese ist eine technische Vorstufe zur Gewinnung von Methacrylsäure.

*Stand der Technik*

Aus einer Arbeit von B.S. Friedman und S.M. Cotton (J.Org.Chem., Band 26, S. 3751-54) ist die Umsetzung von Propylen mit der halben Molmenge CO und der dreifachen Molmenge HF bei 75° C und einem Druck von 38 bar bekannt. Nach der Zersetzung des Reaktionsgemisches mit Wasser wurde Isobuttersäure in 14%iger Ausbeute, bezogen auf Propylen, erhalten. Bezogen auf CO, das in der geringsten Molmenge eingesetzt worden war, betrug die Ausbeute an Isobuttersäure 33%. Zur technischen Gewinnung von Isobuttersäurefluorid ist das Verfahren wegen der geringen Ausbeute nicht geeignet.

In der US-PS Nr. 4303594 wird die Herstellung von Isobuttersäurefluorid aus den gleichen Komponenten beschrieben, jedoch wird bei einer Temperatur von 30° C und einem Druck von 340 bar gearbeitet. Pro Mol Propylen werden 1 Mol CO und 15 Mol HF eingesetzt. Bei einer Verweilzeit von 30 min erhält man Isobuttersäurefluorid in einer Ausbeute von 92%, bezogen auf Propylen. Der hohe Druck in Verbindung mit einer ziemlich langen Verweilzeit machen einen Druckreaktor von sehr erheblicher Grösse und Wandstärke erforderlich.

*Aufgabe und Lösung*

Ziel der Erfindung ist die Auffindung eines kontinuierlichen Verfahrens zur Herstellung von Isobuttersäurefluorid gemäss dem Oberbegriff des Patentanspruchs, das mit niedrigerem Druck und kürzerer Verweilzeit, mithin in einem Druckreaktor von geringerer Grösse und Wandstärke durchgeführt werden kann, und mit hoher Selektivität bzw. Ausbeute abläuft. Weiterhin waren Verfahrensbedingungen zu finden, unter denen Isobuttersäurefluorid in einer zur weiteren Umsetzung zu Isobuttersäure besonders geeigneten Form gewonnen wird und welche zu einer besonders wirtschaftlichen Zusammenfassung beider Reaktionsstufen geeignet sind. Die gesetzten Ziele werden durch das Verfahren gemäss den Patentansprüchen erreicht.

*Ausführung der Erfindung*

Da die Umsetzung erfindungsgemäss unter Druck bei erhöhter Temperatur unter hoher Rückvermischung durchgeführt wird, bedient man sich zweckmässigerweise eines beheizbaren Rührautoklaven aus einem gegenüber Fluorwasserstoff beständigen Werkstoff. Besonders geeignete Werkstoffe, die wenigstens die innere, mit dem Reaktionsgemisch in Berührung stehende Auskleidung des Rührreaktors bilden, sind Aluminium und Nickel-Chrom-Eisen-Legierungen, an denen die genannten Metalle im Verhältnis (30 bis 50) : (20 bis 30) : (18 bis 50) Gew.-% beteiligt sind; vgl. DE-A Nr. 3139653.

Der Druck des Reaktionsgemisches beruht im wesentlichen auf dem CO-Partialdruck und beträgt zwischen 70 und 150 bar, vorzugsweise 80 bis 120 bar, insbesondere 90 bis 100 bar. Der günstigste Temperaturbereich für die Umsetzung beträgt 60 bis 75° C. Die Verweilzeit liegt in der Regel unter 10 min, meistens bei 4 bis 8 min oder sogar darunter.

Die erforderliche hohe Rückvermischung des Reaktionsgemisches wird z.B. durch übliche Flügel- oder Propellerrührer oder Strahldüsen erreicht. Bei ungenügender Rückvermischung kommt es zu einem Konzentrationsanstieg von Propylen an der Einleitungsstelle, was die Bildung von Oligomeren des Propylens nach sich ziehen kann.

Die Reaktionsteilnehmer – Propylen, CO und HF – werden in dem im Anspruch 1 angegebenen Mengenverhältnis kontinuierlich in den Reaktor eingeleitet und eine entsprechend grosse Menge der flüssigen Phase des Reaktionsgemisches abgezogen. CO wird im allgemeinen in der zu Propylen äquivalenten Menge oder einem höchstens geringen Überschuss eingesetzt. Eine günstige HF-Menge liegt etwa bei 10 Mol/Mol Propylen. Die Propylenkonzentration lässt sich nach dem Entspannen einer Probe der flüssigen Phase leicht bestimmen. Ein Teil des Propylens geht stets durch Umsetzung mit HF in Isopropylfluorid über, welches ähnlich wie Propylen an der Reaktion teilnimmt. Da es jedoch weniger zur Oligomerisierung neigt, kann man seine Konzentration im Reaktionsgemisch auf mehr als 1% ansteigen lassen oder sogar direkt Propylfluorid statt Propylen einsetzen.

Von dem eingesetzten Propylen werden unter den erfindungsgemässen Verfahrensbedingungen im allgemeinen mehr als 97% umgesetzt und bis zu 93% in Isobuttersäurefluorid umgewandelt. Daraus ergibt sich eine Selektivität von etwa 96%. Das mit dem Reaktionsgemisch abgezogene Isopropylfluorid wird in den Reaktor zurückgeführt und mindert die Selektivität und Ausbeute nicht.

Das aus dem Reaktionsgefäss abgenommene Gemisch kann entspannt und destillativ aufgearbeitet werden. Die gasförmigen und leichtflüchtigen Anteile, nämlich die Ausgangsstoffe und Isopropylfluorid, werden in den Reaktor zurückgeführt. Das höher siedende Isobuttersäurefluorid, gegebenenfalls im Gemisch mit HF, bleibt zurück

und kann getrennt durch Destillation gereinigt werden.

*Carbonylierung in Gegenwart von organischen Lösungsmitteln*

Bei der oben beschriebenen Arbeitsweise tritt in geringem Umfang eine Oligomerisierung des Propylens ein. Führt man die Carbonylierung unter den oben beschriebenen Bedingungen in Gegenwart einer organischen Flüssigkeit, die mit wasserfreiem Fluorwasserstoff ein Zweiphasensystem bildet, durch, so wird die Oligomerisierung des Propylens stark unterdrückt. Zweckmässig verwendet man eine Flüssigkeit, die spezifisch schwerer als die HF-Phase ist und wenig HF löst. Vorzugsweise beträgt die Löslichkeit des HF weniger als 5 Gew.-% bei 20° C. Isobuttersäurefluorid soll möglichst gut darin löslich sein. Beispiele geeigneter Flüssigkeiten sind aliphatische Halogenkohlenwasserstoffe, bevorzugt solche der Formel

$$C_nH_yHal_{(2n+x-y)}$$

worin «Hal» ein Halogenatom und n eine ganze Zahl von 1 bis 4, x = 0 oder 2, aber x = 2, wenn n = 1, und y eine ganze Zahl vom Wert y = 0 bis n bedeuten. Besonders bevorzugt ist 1,1,2,2-Tetrachloräthan. Aus dem Reaktor kann die flüssige, an HF arme Phase aus einer Beruhigungszone kontinuierlich abgezogen werden. Das darin gelöste Isobuttersäurefluorid kann durch Destillation abgetrennt und die organische Flüssigkeit – gegebenenfalls nach Reinigung eines Teilstromes – in den Carbonylierungsreaktor zurückgeführt werden. Bei dieser Arbeitsweise verbleibt die Hauptmenge des freien HF dauernd im Reaktor. Zweckmässig wird laufend eine kleine Menge der HF-Phase abgezogen und destilliert, um die Anreicherung von Fremdbestandteilen zu verhindern.

Die Menge der pro Mol Propylen einzusetzenden organischen Flüssigkeit wird so gross gewählt, dass nach der Carbonylierung zumindest bei Raumtemperatur eine Phasentrennung zwischen einer hauptsächlich aus Isobuttersäurefluorid und der organischen Flüssigkeit bestehenden flüssigen Phase und einer überwiegend aus Fluorwasserstoff bestehenden flüssigen Phase eintritt. Eine dafür geeignete Menge beträgt z.B. 25 Vol-%. Bei Flüssigkeiten unter 3 Vol-% wird keine ausreichende Phasentrennung erreicht. Mengen über 75 Vol-% erhöhen den erforderlichen Trennungsaufwand, ohne zusätzliche Verfahrensvorteile zu bieten.

*Technische Ausführungsformen*

In Fig. 1 ist eine technische Anlage zur Durchführung des Verfahrens ohne Mitverwendung einer organischen Hilfsflüssigkeit schematisch dargestellt. In den Druckreaktor 1 werden über die Leitungen 2, 3, 4 Propylen, CO und HF kontinuierlich eingespeist. Über die Druckleitung 5 wird das Reaktionsprodukt in die Destillationskolonne 6 geleitet, in der unter Druck HF als Kopfprodukt abdestilliert und zusammen mit nicht umgesetztem Propylen und Isopropylfluorid in den Reaktor 1 zurückgeleitet wird. Aus dem Sumpf der Kolonne 6 wird rohes Isobuttersäurefluorid abgenommen und über ein Entspannungsventil 7 dem Hydrolysereaktor 8 zugeführt, wo es mit Wasser zersetzt wird. Die Hydrolyseprodukte gelangen über die Leitung 9 in die Destillationskolonne 10, aus welcher HF als Kopfprodukt entweicht und mittels des Kompressors 11 in den Druckbereich zurückgeführt wird. Die mit Oligomeren verunreinigte Isobuttersäure gelangt über die Leitung 12 in die Reindestillationskolonne 13, aus der Isobuttersäure als gereinigtes Kopfprodukt abgenommen wird.

Fig. 2 zeigt eine Anlage, bei der die Carbonylierung im Druckreaktor 1 in Gegenwart von Tetrachloräthan durchgeführt wird, welches über die Leitung 20 eingespeist wird. In der Beruhigungszone 22 des Reaktors 1 sammelt sich die weitgehend von HF freie Lösung von Isobuttersäurefluorid in Tetrachloräthan. Die Lösung wird über die Entspannungsleitung 21 abgenommen, das Isobuttersäurefluorid in der Kolonne 23 abdestilliert und Tetrachloräthan aus dem Sumpf über die Leitung 24 in den Reaktor 1 zurückgeführt. Zur Entfernung von Spuren von Oligomeren wird ein Teilstrom des Tetrachloräthans in der Destillationsanlage 25 gereinigt. Die weitere Verarbeitung des Isobuttersäurefluorids in den Anlageteilen 8 bis 13 erfolgt wie in der Anlage gemäss Fig. 1.

Bei der Anlage gemäss Fig. 3 wird die Hydrolyse in Gegenwart des Tetrachloräthans durchgeführt. Das Gemisch aus Isobuttersäurefluorid und Tetrachloräthan wird über die Entspannungsleitung 21 direkt in den Hydrolysereaktor 8 geleitet. Das Hydrolyseprodukt trennt sich im Scheidegefäss 30 in zwei Phasen, die beide Isobuttersäure enthalten. Die obere Phase wird in der Kolonne 10 in HF und Isobuttersäure zerlegt. Diese wird zusammen mit der unteren Phase aus dem Scheidegefäss 30 über die Leitung 31 in die Destillationskolonne 32 geleitet, wo Tetrachloräthan abdestilliert und in den Druckbereich zurückgeführt wird. Die rohe Isobuttersäure wird als Sumpfprodukt aus der Kolonne 32 abgenommen und in der Kolonne 13 reindestilliert.

In den Fig. 1 bis 3 wurden einander entsprechende Anlagenteile mit gleichen Ziffern bezeichnet. Die Materialströme sind wie folgt gekennzeichnet:

| | |
|---|---|
| 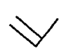 | Propylen |
| CO | Kohlenmonoxid |
| HF | Fluorwasserstoff |
| $H_2O$ | Wasser |
| IBF | Isobuttersäurefluorid |
| IBS | Isobuttersäure |
| Olig. | Oligomere |
| TCE | Tetrachloräthan |
| IPF | Isopropylfluorid |

*Beispiele 1-9*

Die Umsetzungen werden in einem Rührautoklaven aus einer Nickellegierung (Hastalloy C4®) mit 60 ml freiem Reaktorvolumen, ausgerüstet mit

Begasungsrührer, durchgeführt. Die Rührgeschwindigkeit beträgt 600 Upm. Der Autoklav ist mit einer durch den Rührer führenden Gaszuführungsleitung sowie weiteren Zu- und Ableitungen ausgerüstet und kann mittels einer elektrischen Heizung erwärmt werden. CO wird zum Teil über den Begasungsrührer eingeleitet. Die übrigen Komponenten werden über getrennte Leitungen eindosiert.

Propylen, Fluorwasserstoff und CO werden bei einer Reaktortemperatur von 70° C, einem Betriebsdruck von 120 bar, einer mittleren Verweilzeit von 5 min und einem konstanten Propylen/CO-Verhältnis von 1/1,5 Mol umgesetzt. Das Fluorwasserstoff/Propylen-Verhältnis wird von 4/1 bis 20/1 Mol variiert. Im stationären Zustand werden folgende Ausbeuten und Selektivitäten erreicht:

| Beisp. Nr. | $HF/C_3H_6$ (Mol/Mol) | Ausbeute IBF[a)b)c)] (%) | Selektivität[b)c)] (%) | Ausbeute Oligomere[b)] (%) |
|---|---|---|---|---|
| 1 | 4/1 | 7 | 35,9 | 5,7 |
| 2 | 6/1 | 53,9 | 83 | 1,7 |
| 3 | 8/1 | 70,6 | 87,5 | 1,7 |
| 4 | 10/1 | 86,2 | 92,5 | 1,9 |
| 5 | 12/1 | 89,9 | 94,3 | 1,5 |
| 6 | 14/1 | 92,2 | 95,1 | 1,3 |
| 7 | 16/1 | 93,6 | 95,8 | 1,1 |
| 8 | 18/1 | 94,5 | 96,2 | 1 |
| 9 | 20/1 | 95 | 96,5 | 0,6 |

a) IBF = Isobuttersäurefluorid
b) Ausbeuten und Selektivitäten bezogen auf eingesetztes Propylen
c) Gebildetes Isopropylfluorid wird in der Bilanzierung wie nicht umgesetztes Propylen behandelt.

*Beispiele 10-17*

Analog Beispiel 1 werden Propylen, Fluorwasserstoff und CO bei 70° C, einer Verweilzeit von 5 min und einem konstanten Fluorwasserstoff/Propylen/CO-Verhältnis von 10/1/1,5 Mol umgesetzt. Variiert wird der Betriebsdruck von 20 bis 140 bar.

| Beisp. Nr. | Druck (bar) | Ausbeute IBF (%) | Selektivität (%) | Ausbeute Oligomere (%) |
|---|---|---|---|---|
| 10 | 20 | 51,8 | 75,2 | 3,7 |
| 11 | 30 | 58,1 | 78,1 | 2,9 |
| 12 | 40 | 68,4 | 84,7 | 4,5 |
| 13 | 60 | 74,4 | 88,6 | 2,7 |
| 14 | 80 | 81 | 90,6 | 1,8 |
| 15 | 100 | 81,3 | 91,2 | 1,5 |
| 16 | 120 | 86,2 | 92,5 | 1,9 |
| 17 | 140 | 86 | 92,7 | 0,7 |

*Beispiele 18-25*

Analog Beispiel 1 werden Propylen, Fluorwasserstoff und CO im konstanten Molverhältnis von 1/10/1,5 bei einem Betriebsdruck von 60 bar und einer mittleren Verweilzeit von 5 min umgesetzt. Variiert wird die Temperatur von 50 bis 120° C.

| Beisp. Nr. | Temperatur (° C) | Ausbeute IBF (%) | Selektivität (%) | Ausbeute Oligomere (%) |
|---|---|---|---|---|
| 18 | 50 | 61,8 | 88,7 | 1 |
| 19 | 60 | 64,4 | 85,7 | 1,3 |
| 20 | 70 | 74,4 | 88,6 | 2,7 |
| 21 | 80 | 75,9 | 87,0 | 4,2 |
| 22 | 90 | 74,7 | 83,7 | 8,3 |
| 23 | 100 | 75,5 | 81,6 | 12,3 |
| 24 | 110 | 72,5 | 80 | 14,5 |
| 25 | 120 | 72,7 | 78,1 | 17,1 |

*Beispiele 26-29*

Analog Beispiel 1 werden Propylen, Fluorwasserstoff und CO bei 70° C, einem Betriebsdruck von 120 bar und einer Verweilzeit von 5 min in Gegenwart von 1,1,2,2-Tetrachloräthan zur Reaktion gebracht. Konstant gehalten wird das molare Mischungsverhältnis von Fluorwasserstoff/CO/Tetrachloräthan = 10/1,5/3; variiert wird das Verhältnis von Fluorwasserstoff/Propylen von 10/1 bis 10/0,35 Mol.

| Beisp. Nr. | $HF/C_3H_6$ (Mol/Mol) | Ausbeute IBF (%) | Selektivität (%) | Ausbeute Oligomere (%) |
|---|---|---|---|---|
| 26 | 10/1 | 41 | 96,9 | 0,04 |
| 27 | 10/0,7 | 57,5 | 98,7 | <0,04 |
| 28 | 10/0,42 | 75,6 | 98,2 | <0,04 |
| 29 | 10/0,35 | 76,8 | 97,2 | <0,04 |

**Patentansprüche**

1. Verfahren zur kontinuierlichen Herstellung von Isobuttersäurefluorid durch Umsetzung von Propylen und/oder Isopropylfluorid mit der mindestens äquimolaren Menge Kohlenmonoxid und der 10- bis 20-fachen Molmenge Fluorwasserstoff bei erhöhtem Druck, dadurch gekennzeichnet, dass die Reaktion bei einer Temperatur zwischen 40 und 90° C und bei einem Druck zwischen 70 und 150 bar unter hoher Rückvermischung durchgeführt wird und dass die kontinuierliche Zufuhr der Ausgangsstoffe so eingestellt wird, dass die Verweilzeit des Reaktionsgemisches unter den genannten Reaktionsbedingungen nicht mehr als 20 min beträgt und die Konzentration an freiem Propylen nicht über 1 Gew.-% (bezogen auf die flüssige Phase) ansteigt.

2. Verfahren zur Herstellung von Isobuttersäurefluorid in einer ersten Reaktionsstufe gemäss Anspruch 1 und zur weiteren Umsetzung des Isobuttersäurefluorids zu Isobuttersäure durch Hydrolyse mit Wasser in einer zweiten Reaktionsstufe, dadurch gekennzeichnet, dass die flüssige Phase des gemäss Anspruch 1 erhaltenen Reaktionsgemisches in der zweiten Reaktionsstufe mit der zur vollständigen Hydrolyse des gebildeten Isobuttersäurefluorids erforderlichen Wassermenge versetzt und nach oder während der Hydrolyse die niedriger als Isobuttersäure siedenden Bestandteile des Hydrolysegemisches destillativ abgetrennt und überwiegend in das Reaktionsgemisch der ersten Reaktionsstufe zurückgeleitet werden.

3. Verfahren nach den Ansprüchen 1 oder 2, dadurch gekennzeichnet, dass die Umsetzung in der ersten Stufe in Gegenwart einer organischen Flüssigkeit durchgeführt wird, in welcher bei der Umsetzungstemperatur Fluorwasserstoff schwer und Isobuttersäurefluorid gut löslich ist, wobei die Menge der organischen Flüssigkeit so gross gewählt wird, dass nach der Carbonylierung zumindest bei Raumtemperatur eine Phasentrennung eintritt.

4. Verfahren nach Anspruch 3, dadurch gekennzeichnet, dass als organische Flüssigkeit ein aliphatischer Halogenkohlenwasserstoff eingesetzt wird.

5. Verfahren nach Anspruch 4, dadurch gekennzeichnet, dass ein Halogenkohlenwasserstoff der Formel

$$C_nH_yHal_{(2n+x-y)}$$

worin «Hal» ein Halogenatom und n eine ganze Zahl von 1 bis 4, x = 0 oder 2, aber x = 2, wenn n = 1, und y eine ganze Zahl vom Wert y = 0 bis n bedeuten, eingesetzt wird.

6. Verfahren nach Anspruch 5, dadurch gekennzeichnet, dass als Halogenkohlenwasserstoff 1,1,2,2-Tetrachloräthan eingesetzt wird.

**Claims**

1. Process for continuously preparing isobutyryl fluoride by reacting propylene and/or isopropylfluoride with an at least equimolar quantity of carbon monoxide and 10 to 20 times the molar quantity of hydrogen fluoride under elevated pressure, characterized in that the reaction is carried out at a temperature of between 40 and 90° C and under a pressure of between 70 and 150 bar with high remixing and the continuous supply of starting materials is adjusted so that the retention time of the reaction mixture under the reaction conditions specified is not more than 20 minutes and the concentration of free propylene does not increase above 1 % by weight (based on the liquid phase).

2. Process for preparing isobutyryl fluoride in a first reaction step as claimed in Claim 1 and for further reacting the isobutyryl fluoride to form isobutyric acid by hydrolysis with water in a second reaction step, characterized in that the liquid phase of the reaction mixture obtained according to Claim 1 is mixed, in the second reaction step, with the quantity of water necessary for total hydrolysis of the isobutyryl fluoride formed and after or during the hydrolysis the components of the hydrolysis mixture which have a lower boiling point than isobutyric acid are removed by distillation and predominantly recycled into the reaction mixture of the first reaction step.

3. Process as claimed in Claim 1 or 2, characterized in that the reaction in the first step is carried

out in the presence of an organic liquid in which hydrogen fluoride is poorly soluble and isobutyryl fluoride is readily soluble at the reaction temperature, the quantity of organic liquid being selected so that after carbonylation phase separation occurs at least at ambient temperature.

4. Process as claimed in Claim 3, characterized in that an aliphatic halohydrocarbon is used as the organic liquid.

5. Process as claimed in Claim 4, characterized in that a halohydrocarbon of formula

$$C_nH_yHal_{(2n+x-y)}$$

wherein 'Hal' is a halogen atom and n is an integer from 1 to 4, x = 0 or 2, but x = 2 if n = 1, and y represents an integer of the value y = 0 to n, is used.

6. Process as claimed in Claim 5, characterized in that 1,1,2,2-tetrachloroethane is used as the halohydrocarbon.

## Revendications

1. Procédé pour la préparation en continu de fluorure d'acide isobutyrique par réaction de propylène et/ou de fluorure d'isopropyle avec la quantité au moins équimolaire d'oxyde de carbone et 10 à 20 fois la quantité molaire de fluorure d'hydrogène, sous pression élevée, caractérisé en ce que la réaction est conduite à une température comprise entre 40 et 90° C et sous une pression comprise entre 70 et 150 bar, avec fort remélangeage, et en ce que l'introduction continue des substances de départ est réglée de telle manière que la durée de séjour du mélange réactionnel dans les conditions de réactions mentionnées ne s'élève pas à plus de 20 min et que la concentration de propylène libre ne dépasse pas 1 % en poids (sur la base de la phase liquide).

2. Procédé pour la préparation de fluorure d'acide isobutyrique dans un premier stade réactionnel selon la revendication 1 et pour la transformation ultérieure du fluorure d'acide isobutyrique en acide isobutyrique par hydrolyse par l'eau dans un second stade réactionnel, caractérisé en ce que la phase liquide du mélange réactionnel obtenu selon la revendication 1 est additionnée, dans le second stade réactionnel, de la quantité d'eau nécessaire pour l'hydrolyse complète du fluorure d'acide isobutyrique formé et, après ou pendant l'hydrolyse, les constituants du mélange d'hydrolyse dont le point d'ébullition est plus bas que celui de l'acide isobutyrique sont séparés par distillation et sont renvoyés en majeure partie dans le mélange réactionnel du premier stade réactionnel.

3. Procédé selon la revendication 1 ou 2, caractérisé en ce que la réaction dans le premier stade est conduite en présence d'un liquide organique dans lequel, à la température de réaction, le fluorure d'hydrogène est peu soluble et le fluorure d'acide isobutyrique est bien soluble, la quantité du liquide organique étant choisie suffisamment grande pour qu'à la suite de la carbonylation, il se produise une séparation de phases, au moins à la température ambiante.

4. Procédé selon la revendication 3, caractérisé en ce qu'un hydrocarbure aliphatique halogéné est utilisé comme liquide organique.

5. Procédé selon la revendication 4, caractérisé en ce qu'il est utilisé un hydrocarbure halogéné de formule

$$C_nH_yHal_{(2n+x-y)}$$

dans laquelle «Hal» représente un atome d'halogène et n est un nombre entier de 1 à 4, x = 0 ou 2, mais x = 2 lorsque n = 1, et y est un nombre entier de 0 à n.

6. Procédé selon la revendication 5, caractérisé en ce que le 1,1,2,2-tétrachloréthane est utilisé comme hydrocarbure halogéné.

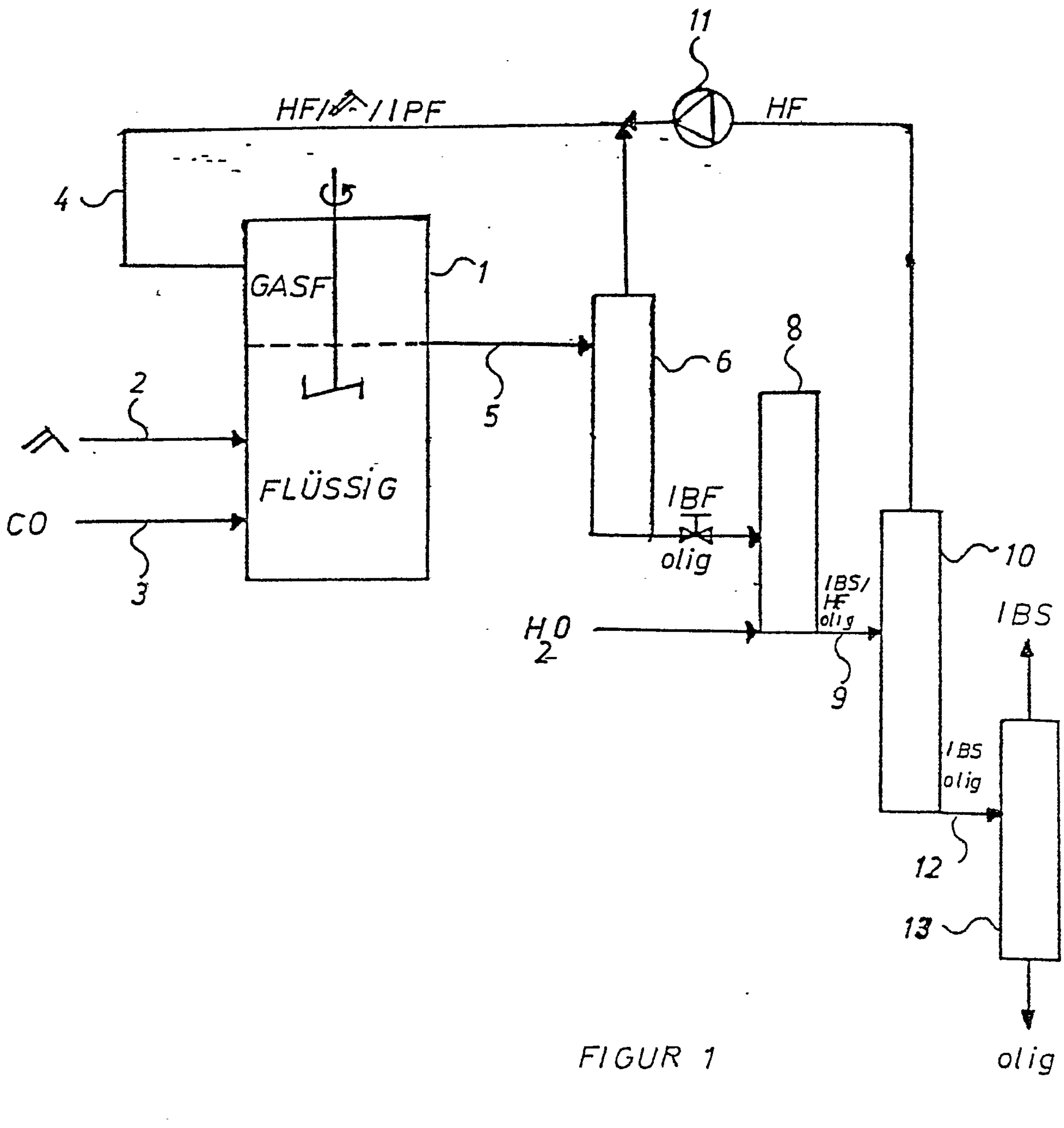
11
HF/
/IPF
HF
4
1
GASF
6
8
2
5
FLÜSSIG
CO
3
IBF
olig
10
IBS/
HF
olig
IBS
H2O
9
IBS
olig
12
13
olig

FIGUR 1

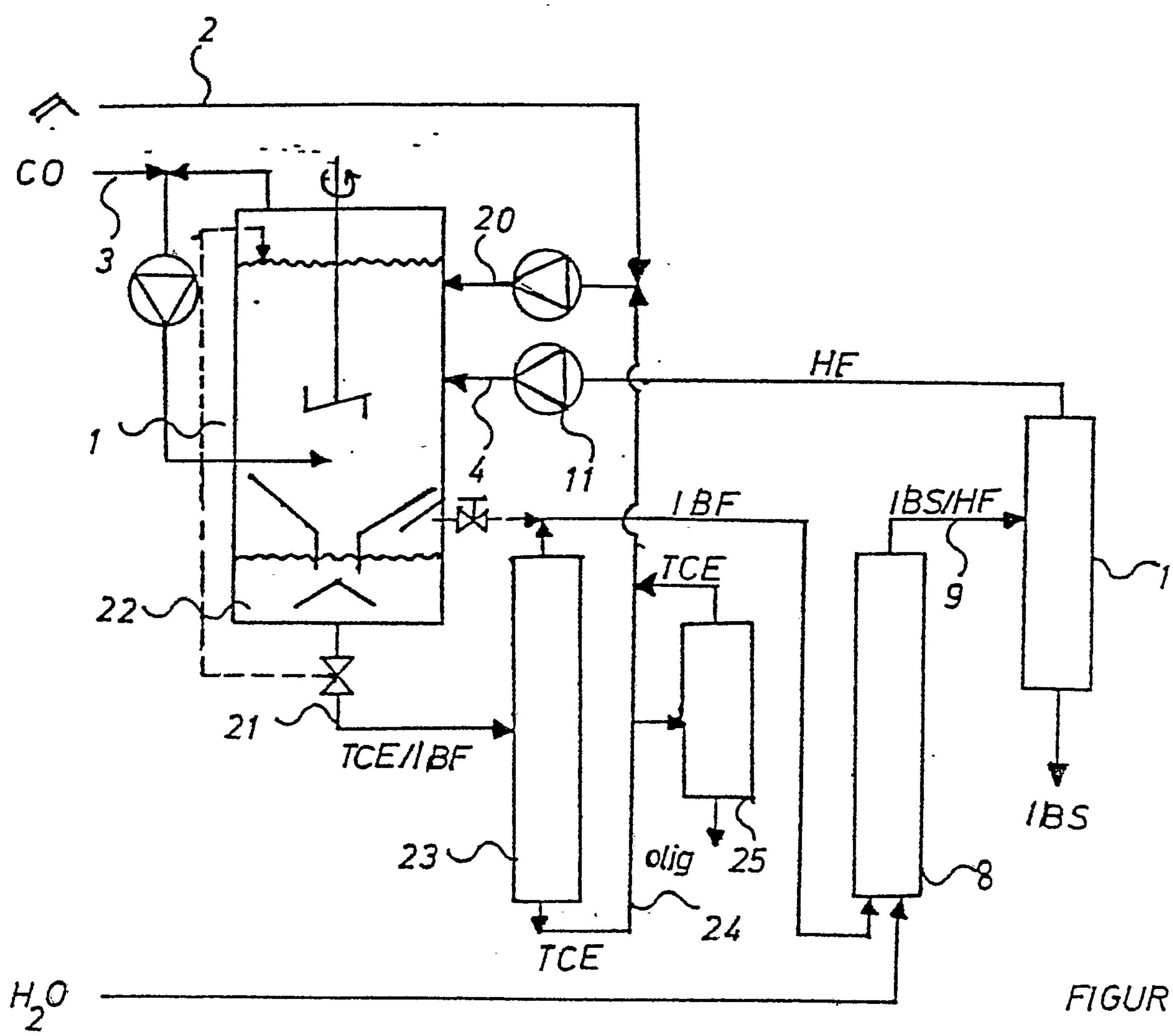
2
CO
3
20
1
4
11
HF
IBF
IBS/HF
TCE
9
10
22
21
TCE/IBF
IBS
23
olig
25
8
24
TCE
$H_2O$

FIGUR 2

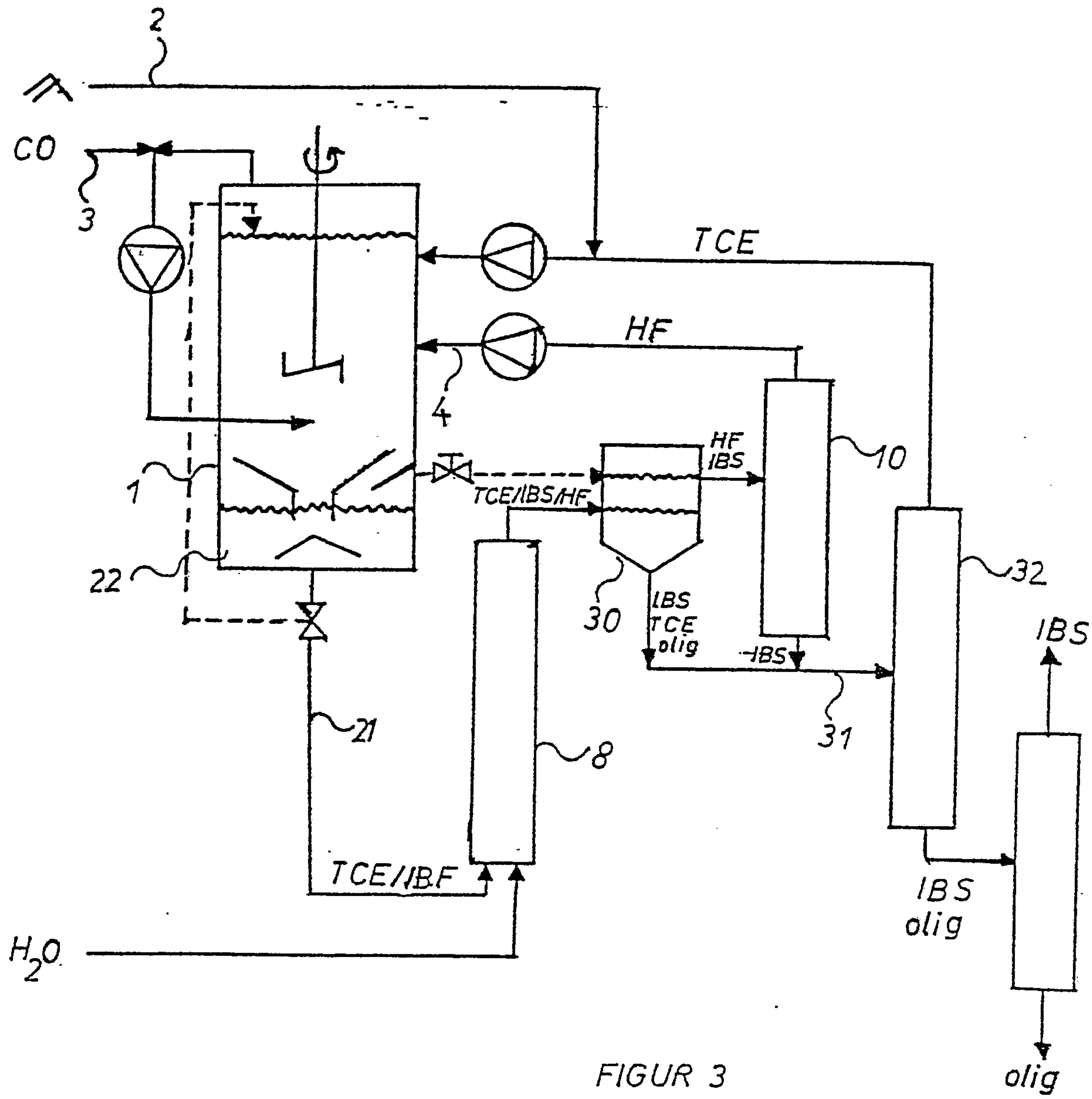
2
CO
3
TCE
HF
4
1
22
TCE/IBS/HF
HF
IBS
10
30
IBS
TCE
olig
IBS
31
32
IBS
21
8
TCE/IBF
IBS
olig
H2O
olig

FIGUR 3